Europäisches Patentamt

European Patent Office

Office européen des brevets

(19)

(11) Publication number: **0 162 977 B1**

(12) **EUROPEAN PATENT SPECIFICATION**

(45) Date of publication of patent specification: **20.05.92**  (51) Int. Cl.⁵: **G01N 33/53**

(21) Application number: **84304774.7**

(22) Date of filing: **12.07.84**

The file contains technical information submitted after the application was filed and not included in this specification

(54) **Enzyme immunoassay for cancer procoagulant.**

(30) Priority: **02.05.84 US 606330**

(43) Date of publication of application:
**04.12.85 Bulletin 85/49**

(45) Publication of the grant of the patent:
**20.05.92 Bulletin 92/21**

(84) Designated Contracting States:
**AT BE CH DE FR GB IT LI LU NL SE**

(56) References cited:
**EP-A- 0 145 373**
**FR-A- 2 381 058**
**GB-A- 2 067 286**
**US-A- 4 379 839**

**CHEMICAL ABSTRACTS, vol.100, no.9,27th February 1984, Columbus, Ohio, US; D.E. SCHUMM et al. "Absence of the cancer-associated factor with a molecular weight of 60.000 from the plasma of patients with a spectrum of nonneoplasticconditions"**

**Gordon et al, Proc. Ann. Meet. Am. Soc. Clin. Oncol. 3:6, 1984**

Gordon et al., Clin. Res. 32:415, 1984

(73) Proprietor: **University Research Corporation**
**1305 University Avenue**
**Boulder, Colorado 80309(US)**

(72) Inventor: **Gordon, Stuart G., Dr.**
**20 South Ash**
**Denver, CO 80222(US)**

(74) Representative: **Allam, Peter Clerk et al**
**LLOYD WISE, TREGEAR & CO. Norman House 105-109 Strand**
**London WC2R 0AE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 162 977 B1

Rank Xerox (UK) Business Services
(3.08/2.19/2.0)

## Description

This invention relates to a monoclonal antibody, to a method for producing the antibody, to a diagnostic of the diagnostic reagent in a highly sensitive and specific immunoassay of a proteolytic procoagulant enzyme in biological samples, for example biological fluids such as human and animal serum, plasma, tissue extracts and histologic sections. The presence of this enzyme is indicative of malignant disease, and the immunoassay described may be used as part of a diagnostic test for cancer.

For years, investigators have sought to identify substances that are unique to tumor cells for use as diagnostic markers of cancer. In 1970, Bubenek et al, reporting in Int. J. Cancer 5:310 (1970), demonstrated that serum from cancer patients contained antibodies that bound to tumor cell surface antigens. Subsequently, many antigens were reported found on the surface of human melanoma and on other neoplastic cells. Some of these antigens have been identified with normal fetal tissue, for example antigens common to human colonic carcinomas and fetal gut epithelium. Since fetal tissue is comprised of undifferentiated cells, and neoplastic cells are "dedifferentiated cells", the accepted working hypothesis for tumor antigens or oncofetal antigens is that certain proteins are expressed by cells in their undifferentiated state, and the expression of these proteins is suppressed when undifferentiated cells become differentiated into normal cells. If these normal cells become dedifferentiated during the malignant transformation process, the genetic information is derepressed and these tumor antigens are again expressed. An alternative school of thought suggests that partially differentiated "stem" cells of normal adult tissues are held in their partially differentiated state by carcinogenic agents, and these partially differentiated cells are able to multiply in an uncontrolled fashion. In either case, malignant transformation is recognized to result in the genetic expression of protein antigens that are associated with the undifferentiated cell.

Many tumor antigens have been identified and characterized during the past 20 years. Most notable among these are carcinoembryonic antigen (CEA), alpha-fetoprotein (AFP) and acute lymphoblastic leukemia associated antigen (cALLA). Carcinoembryonic antigen was first described by Gold and Friedman (J. Exp. Med. 121:439 (1965)) who detected it in colon carcinomas and fetal gastrointestinal tract tissue. CEA is a high molecular weight (180-200 kd) protein that is composed of 45-57% carbohydrate and 30-46% protein. CEA, or CEA-like material, is produced by a variety of mucin producing normal epithelial tissues including normal colon. In addition, a variety of nonmalignant disorders are associated with elevated plasma levels of CEA including peptic ulcers (10%), pancreatitis (27%), inflammatory bowel diseases (15-40%), hepatic disease, including hepatitis, jaundice, biliary tract disease and cirrhosis (20-80%). In addition, CEA levels have been widely studied as predictive of various types of malignant disease including tumors of the GI tract (30%), gastric cancer (72%), pancreatic cancer (88%), breast cancer (24%), lung and respiratory tract tumors (30%) and gynecological tumors (10%) (J.D. Beatty, et al, Prog. Clin. Cancer 8:9 (1982)). There appears, however, to be no close correlation between the type and size of the tumor and the plasma CEA level; although patients with metastatic disease and stage 4 cancer appear to have higher levels than patients without metastatic disease or early stage malignancy. CEA levels have been helpful in follow-up studies of breast and colorectal cancer; if the tumor is CEA-positive, monitoring CEA levels has been found to provide important information as to the efficacy of therapeutic treatment of the disease.

Alpha-fetoprotein is a protein of about 70 kd with amino acid composition similar to that of serum albumin, is produced by fetal liver and can be detected in amniotic fluid and maternal serum. Alpha-feto protein has also been found to be present in hepatocellular carcinoma. AFP is elevated in the serum of about 80% of patients with liver tumors, almost all those with teratocarcinomas, 15% of patients with gastric carcinoma, 3% of patients with colorectal carcinoma, 24% of patients with hepatic carcinoma, and 25% of patients with biliary tract carcinomas (see Ruddon, Semin. Oncol. 9:416 (1982) and McIntire, et al, Cancer Res. 35:991 (1975)). AFP rises as a function of a variety of insults to the liver and in pregnancy. In spite of the apparent high predictive value of AFP for cancer and a small number of non-cancer diseases that appear to have elevated AFP levels, the overall value of AFP as a tumor marker is low.

Recently, acute lymphoblastic leukemia antigen (cALLA) has been studied to determine its effectiveness in identifying acute lymphoblastic leukemia patients (see Ritz, et al, Nature 283:583 (1980)). Although initial studies suggested that cALLA was specific for acute leukemic cells, more recently the antigen has been found on a variety of normal cell types including normal kidney epithelium and melanomas. Its efficacy as a diagnostic marker of leukemia is yet to be firmly established.

There are a large number of other tumor associated antigens that have been studied for their potential as tumor markers. Tumor markers to the following human cancers are reviewed by Hellstrom et al, Springer Semin Immunopathol 5:127 (1982): melanoma, neuroblastoma, glioma, colorectal carcinoma, gastric carcinoma, mammary carcinoma, brachiogenic carcinoma, pancreatic carcinoma, ovarian carcinoma, Wilms' tumor, renal cell carcinoma, transitional cell carcinoma of the bladder, osteogenic sarcoma, carcinoma of the uterine cervix and lymphoma. These tumor antigens are poorly characterized and have not been carefully tested for their ability to diagnose clinical cancer. None of them have proven value as tumor markers or as diagnostic indicators of cancer.

Cancer procoagulant was originally identified during studies to seek a substance that initiated the abnormal blood coagulation associated with malignant disease. This protein has been purified to homogeneity and characterized. It is a cysteine protease that initiates coagulation by directly activating factor X in the coagulation cascade.

Cancer procoagulant appears to have no carbohydrate ( 1 mole sialic acid, or hexose/mole of cancer procoagulant), it has a molecular weight of about 68,000 from all species that have been examined, including mouse, rabbit, and human. Cancer procoagulant is eluted from a 1.5 M agarose gel filtration column in the void volume indicating that the protein aggregates into a very high molecular weight complex ( 1.5 x 10$^6$ daltons) during this type of gel filtration procedure. It is a single polypeptide protein with the molecular weight of 68 kd and an isoelectric point of about 4.8. It is inhibited by mercury and iodoacetamide, properties that are characteristic of cysteine proteases. To determine the distribution of cancer procoagulant activity in various tumor types, a variety of human tumor extracts and some of their normal tissue counterparts and a variety of serum-free culture media from transformed cells and media from their normal cell counterparts were examined. It was found that cancer procoagulant activity existed in extracts of malignant cells and tissue culture medium from transformed cells, but not in extracts of normal tissue and serum-free medium from normal cells and culture.

The discovery of cancer procoagulant was first reported in 1975 (Gordon et al (1975) Thromb. Res. 6:127-138). Later publications described the isolation and characterization of the protein (see e.g. Gordon et al (1978) Cancer Res. 38:2467-2472; Gordon et al (1979) J. Natl. Cancer Inst. 62:773-776; Gordon et al (1979) Proc. Am. Assoc. Cancer Res. 20:177; Gordon et al (1979) Clin. Res. 27:54; Gordon et al (1981) J. Clin. Invest. 67:1665-1671; Gordon et al (1981) J. Histochem. Cytochem. 29:457-463; Gordon et al (1981) Clin. Res. 29:65A; Gordon et al (1982) Thromb. Res. 26:379-387; Gordon et al (1984) Proc. Ann. Meet. Am. Soc. Clin. Oncol. 3:6 and Gordon et al (1984) Clin. Res. 32:415).

In two abstracts published in March and May, 1984, respectively, it was suggested by Gordon et al that a monoclonal antibody to cancer procoagulant might serve as a tumour marker in an enzyme immunoassay for the diagnosis of cancer (Gordon et al (1984) Proc. Ann. Meet. Am. Soc. Clin. Oncol. 3:6; Gordon et al (1984) Clin. Res. 32:415).

The present invention is predicated on the discovery of a method whereby an immunologically active monoclonal antibody to cancer procoagulant, useful in a diagnostic reagent for the detection of cancer in a patient, may be obtained.

It is well know that monoclonal antibodies against a specific antigen can be obtained by the classical fusion technique of Köhler and Milstein (Nature (1975) 256:495). Applying this classical technique to the production of monoclonal antibody to cancer procoagulant leads to a method which comprises the steps of:

(a) immunizing an animal with cancer procoagulant antigen;

(b) fusing spleen cells from the immunized animal with myeloma cells to form hybrid cells;

(c) culturing the hybrid cells in a selective medium;

(d) testing for the presence of the desired antibody;

(e) cloning cells producing the desired antibody; and

(f) isolating the desired antibody.

However, this method does not lead, in the case of cancer procoagulant, to a monoclonal antibody which is immunologically active and therefore of any value.

In accordance with the present invention we have discovered that, in order for immunologically active monoclonal antibodies to cancer procoagulant to be obtained, it is necessary that medium from the cells producing the desired antibody, as obtained in step (e) above, be subjected to the step of dissociating antibody from cancer procoagulant antigen produced by the hybrid cells used for the production of the antibody prior to isolating the antibody, as in step (f) above.

Thus, in one aspect, the present invention provides a method for producing immunologically active monoclonal antibody to cancer procoagulant which comprises steps (a) to (f) as set forth above and is characterized by the dissociation step, performed between steps (e) and (f), which has been defined in the preceding paragraph.

In another aspect, the present invention provides dissociated immunologically active monoclonal antibody to cancer procoagulant, said monoclonal antibody being substantially free of procoagulant activity.

Further, the present invention provides a reagent for detection of cancer procoagulant comprising immunologically active monoclonal antibody produced by the method of this invention.

Still further, the present invention provides a method for detecting for the presence of cancer procoagulant in a biological sample of a patient, which comprises contacting the biological sample with a reagent which contains immunologically active monoclonal antibody of this invention, as defined above, or produced by the method of this invention, and detecting the presence or absence of an immunological reaction. The biological sample may be, for example, serum, plasma, tissue extracts, urine and histological sections.

This method for detecting for the presence of cancer procoagulant has high sensitivity and specificity, and therefore is a valuable technique for use in the diagnosis of cancer.

The following description is presented in order to provide a thorough understanding of the subject matter and the experimental procedures used in the present application.

Example 1

Materials:

A 0.5M stock solution of diisopropylfluorophosphate (DFP) was prepared in dry isopropyl alcohol and diluted 1:100 in samples for DFP treatment. Crude phospholipid was obtained by chloroform-methanol (3:1) extraction (see Canadian Journal of Biochemical Physiology; 37:911; 1959) of Rabbit V2 carcinoma. Rabbit brain thromboplastin and Russell's viper venom were used as standards in the coagulation system and as representative procoagulants for comparison of the enzymatic properties with cancer procoagulant. Crude rabbit brain cephalin, veronal buffer and bovine plasma deficient in factors VII and X were commercially purchased. Four parts of fresh bovine plasma were collected in one part 3.8% sodium citrate after discarding the first blood through the needle and centrifuged twice for 5 minutes at 1600 xg to remove blood cells.

Example 2

Preparation of Separation Columns:

Benzamidine-Sepharose affinity resin was prepared by coupling $\epsilon$-amino caproic acid to cyanogen bromide activated Sepharose and 100 mg of p-aminobenzamidine was coupled to 2 g of hexanoylsepharose with soluble carbodiimide for 24 hours while maintaining the pH at 4.75. After thorough washing of the resin with distilled water, at 1 x 11 cm column was packed and equilibrated with a 10mM veronal buffer (pH 7.8) containing 50 mM NaCl and 1 mM EDTA. The flow rate of this column was 0.5ml/min.

A gel filtration column (1.5 x 100 cm) was packed with 1.5M agarose and equilibrated with 10 mM veronal buffer at pH 7.8 containing 0.5 mg/ml of crude phospholipid and then washed free of excess phospholipid with 10mM veronal buffer (pH 7.8). The flow rate of this column was about 1 ml/min.

A phenyl-Sepharose® hydrophobic chromatography column (1 x 5 cm) was equilibrated in 10 mM veronal buffer (pH 7.8) containing 0.5 mg/ml of crude phospholipid, washed free of excess phospholipid with the veronal buffer and finally equilibrated with 10 mM veronal buffer (pH 7.8). The column was used at a flow rate of 0.2 ml/min.

A p-chloromercurialbenzoate-agarose affinity resin was prepared by equilibration of the resin in 25mM 2[N-morpholine]-ethane sulfonic acid buffer at pH 6.8. The equilibrated resin was packed in a 1 x 10 cm column and run at a flow rate of about 1.0 ml/minute.

All column chromatography elutions were monitored at 280 nm, and the protein content of aliquots of pooled fraction samples from each step of the purification was routinely determined.

Example 3

Source of Cancer Procoagulant:

V2 carcinoma cells were injected into the thigh muscle of young (2 kg) New Zealand white rabbits and the animal's weight and tumor size were monitored bi-weekly until the animal's weight began to decline and the tumor was large. The tumor was removed surgically prior to the animal's death. Tumors ranged in size from 50 to 200 g. Tumor tissue was cut into 0.5 to 1 cm thick slices to increase surface area and extracted by placing it in 3 changes of 20 mM veronal buffer (pH 7.8) for 3 hours each. The Factor VII depleted bovine plasma was used to determine the dependence of the procoagulant activity on Factor VII, using Russell's viper venom and rabbit brain thromboplastin as positive and negative controls, respectively. DFP

4

sensitivity and activity in Factor VII-depleted bovine plasma were two criteria used throughout the purification procedure as identifying characteristics of the enzyme and to distinguish cancer procoagulant from normal tissue thromboplastin.

A two-stage coagulation assay was used to determine the direction activation of pure bovine Factor X by cancer procoagulant. The first stage contained 0.64 $\mu$g of purified bovine Factor X in 0.5 ml of 50 mM Tris-HCL buffer (pH 7.8) containing 10 mM $CaCL_2$ and 0.15 M NaCl, 10 $\mu$g crude rabbit brain cephalin in 20 $\mu$l saline and from 5 to 10 ng of purified cancer procoagulant such that the ratio of cancer procoagulant to Factor X was from 1:60 to 1:128. A portion of the purified samples was adjusted to 5 mM DFP, incubated at 25°C for 30 minutes and added to the first stage of the assay. Partially purified Russell's viper venon standard was diluted 1:100,000 with saline and used in a ratio of 1:320,000 with Factor X. Aliquots (100 ml) of the first stage reaction mixture were taken at various time intervals, including 0 time, and assayed for Factor Xa activity in the second stage by mixing with 100 $\mu$l of bovine plasma depleted of Factor VII and Factor X and 100 $\mu$l of 20 mM $CaCl_2$. If any Factor Xa was detected in the Factor X samples, they were treated with 25 mM DFP to inactivate the Factor Xa and then dialyzed to remove the residual DFP prior to its use.

To visually demonstrate the direct proteolytic activation of Factor X by cancer procoagulant, 19 $\mu$g of purified bovine Factor X in 15 $\mu$l of 50 mM Tris-HCl buffer (pH 7.8) containing 10 mM $CaCl_2$ and 0.15 M NaCl, 2.5 $\mu$g of crude rabbit brain cephalin in 5 $\mu$l of saline was incubated with .38 $\mu$g of cancer procoagulant. In the control experiment, 13.6 $\mu$g of Factor X was incubated with 90 pg of Russell's viper venom in the same reaction conditions. Aliquots (10 $\mu$l) were removed at 30 seconds, 5 hours., and 15 hours, 2 $\mu$l of 0.05 M EDTA were added, and the aliquot was added to $\frac{1}{4}$ volume of sample buffer without $\beta$-mercaptoethanol for analysis on a 12.5% sodium dodecylsulfate-polyacrylamide gel electrophoresis.

Example 4

Separation Procedure:

Step 1: The concentrated crude tumor extract was applied to the benzamidine affinity chromatography column and unbound protein was washed from the column with 10 mM veronal buffer (pH 7.9) containing 50 mM NaCl and 1 mM EDTA. The bound protein was eluted with 1.0 M propionic acid. The acid elute fractions were brought to pH 7.5 immediately with 4N NaOH or they were collected in an equal volume of 0.5 M veronal buffer (pH 8.0) to partially neutralize the propionic acid and then adjusted to pH 7.5 with NaOH. All the DFP sensitive, Factor VII independent procoagulant activity was recovered in the acid eluate. The fractions containing procoagulant were pooled, concentrated about 20 fold on an ultrafiltration (PM-10) membrane and dialyzed against 10 mM veronal buffer (pH 7.8) to remove sodium propionate. The quantitative recovery of the procoagulant activity was greater than 100%, probably due to the removal of protease inhibitors present in the tumor extract.

Step 2: The concentrated, dialyzed acid elution peak from the affinity column with a protein concentration of from 6 to 10 mg/ml, was applied to a 1.5 M agarose gel filtration column. The column was eluted with 10 mM veronal buffer (pH 7.8); the elution profile had four major protein peaks with the high molecular weight, peak containing most of the DFP sensitive procoagulant activity. Fractions from the major procoagulant peak were pooled and concentrated about 5-fold by ultrafiltration and carried to the next step in the purification sequence. The recovery of activity from this step was frequently greater than 100% probably due to removal of additional inhibitors.

Step 3: The concentrated procoagulant peak was applied to the benzamidine affinity resin, a small amount of unbound protein was washed from the column with 10 mM veronal buffer (pH 7.8) containing 50 mM NaCl and 1 mM EDTA, and protein adsorbed to either the resin or to bound proteins was removed by washing the column with 0.1% Triton® X-100 in 10 mM veronal buffer (pH 7.8). The Triton® X-100 was cleared from the column with the initial veronal buffer, 30 mls of 0.05 M propionic acid was used to elute weakly bound proteases, including some procoagulant activity, and then 40 mls of 0.5 M propionic acid was used to remove the remaining bound proteins. The acid eluates were either adjusted to pH 7.5 immediately, or collected in 0.5 M veronal buffer to partially neutralize the propionic acid as described in Step 1. The samples were dialyzed against 10 mM veronal buffer to remove the sodium propionate, and concentrated by ultrafiltration. No procoagulant activity was recovered in the unbound protein sample and little or no activity was recovered with the protein eluted by Triton X-100. About 30% of the procoagulant activity was recovered in the 0.05 M acid eluate; the remaining 70% was in the 0.5M acid eluate which was carried to the next step of the purification. Although the acid eluate from this purification step formed a single immunoprecipitin band on immunoelectrophoresis against an antibody to partially purified cancer procoagulant is contained 3 to 4 protein impurities when analyzed by sodium dodecylsulfate-polyacrylamide gel electrophoresis.

Step 4: About 1.0 ml of the sample eluted from the second benzamidine affinity column was applied to the PCMB affinity resin in 20 mM veronal buffer (pH 7.7) and washed onto the column with 40 ml of MES buffer (ph 6.8), 40 ml of 1 M urea and 1% Tween® 20 in MES buffer, 20 ml MES buffer to clear the urea and Tween® from the column, 35 ml of 0.1 mM dithiothreitol, 35 ml of 5 mM dithiothreitol, 35 ml of 10 mM dithiothreitol, and finally 35 ml of 100 mM dithiothreitol to strip the column of residual protein. Protein eluted from the column was continuously monitored at 280 nm. The protein peaks were collected separately and concentrated about 10 fold on an ultrafiltration membrane and dialyzed against MES buffer to remove the dithiothreitol. Protein was eluted in each of the washes; a small amount of procoagulant activity was eluted in 0.1 mM DTT with the major peak of activity in the 5 mM eluate.

Step 5: The concentrated, dialyzed procoagulant sample from the PCMB-Sepharose® column was applied to a phenyl-Sepharose® hydrophobic affinity column in 10 mM veronal buffer (pH 7.8) containing 10 μg/ml of crude phospholipid. The sample was allowed to equilibrate with the column for 10 minutes and then unbound protein was eluted with 10 mM veronal buffer (pH 7.8). Procoagulant was eluted with 10% dimethyl sulfoxide in veronal buffer, concentrated by ultrafiltration and dialyzed free of dimethyl sulfoxide against veronal buffer. Although about 20% procoagulant activity was recovered in the veronal buffer, dimethyl sulfoxide eluate generally contained most of the remaining 80% of the activity and was a single protein band by sodium dodecylsulfate-polyacrylamide gel electrophoresis.

The separation procedure uses a number of modifications not previously found, or expected, in the conventional separation of proteins:

The second step of the purification procedure utilized the observation that the procoagulant enzyme aggregated when concentrated to more than 2 mg of protein per ml of sample. This permits it to be resolved from other proteins with molecular weights less than 150,000, a molecular weight cut off that is common for most serine proteases. Following gel filtration column chromatography, the benzamidine-affinity chromatography step was repeated, but adsorbed impurities were removed with a nonionic detergent (0.1% Triton® X-100) and a low level of propionic acid (0.05 M) was used to elute weakly bound proteases, including some cancer procoagulant, before the remaining proteases were stripped from the column with 0.5 M propionic acid. A final hydrophobic affinity chromatography step resulted in highly purified protein. The overall purification and recovery were impossible to calculate accurately because inhibitors present in the crude extract masked the procoagulant activity and the instability of the procoagulant resulted in slow but continuous loss of activity. However, the final product of the purification sequence appears to be homogeneous cancer procoagulant enzyme. Also, phospholipid was used to preequilibrate the columns, and routinely added to samples during the purification, including the purified enzyme, because it was demonstrated to improve both the stability and activity of the procoagulant.

Example 5

Gel Electrophoresis and Electrofocusing:

Analytical polyacrylamide slab gel electrophoresis was carried out with 10% or 12.5% gels at pH 8.9. Aliquots of samples (4 parts) were added to 1 part of a sample buffer solution containing 10% $\beta$-mercaptoethanol, 10% sodium dodecylsulfate, 40% glycerol and 0.01% pyronin Y in 0.125 M Tris-base. Non-reduced samples were prepared in the same sample buffer with $\beta$-mercaptoethanol omitted. The samples were heated for 2 minutes in boiling water and applied to the gel. The molecular weight of the pure procoagulant was estimated by determining the electrophoretic migration of proteins with known molecular weight.

Analytical polyacrylamide gel isoelectric focusing was carried out with precast 4% LKB gels according to the standard LKB procedure. The isoelectric point of the pure procoagulant was determined both by the location of proteins of known isoelectric point, and by determining the pH gradient by measuring the pH of 0.1 M KC1 solution containing 0.5 cm gel slices.

Purified cancer procoagulant appeared as a pair of protein bands on a wide pH range (pH 3.5 to 9.5) analytical polyacrylamide isoelectric focusing gel at a pI of about 4.8 and 4.9. Analysis on a narrow pH range (pH 4.0 to 6.5) gel resolved the pI 4.9 protein band into 2 protein bands, suggesting that there were 3 isozymes of cancer procoagulant.

The amount of procoagulant activity in milliequivalents of rabbit brain thromboplastin (meq RBT) and protein (mg) content of the purification sequence for a representative purification is tabulated in the following Table I. The specific activity (SA, meq RBT/mg protein), the recovery of activity (%) and the increase in specific activity (purification) were calculated from data obtained. It is believed that recoveries greater than 100% are probably due to the removal of procoagulant inhibitors during purification.

Table I

| The Results of a Cancer Procoagulant Purification Sequence | | | | | |
|---|---|---|---|---|---|
| Sample | Total Activity (meq RBT) | Protein (mg) | SA (meq RMB/mg) | %Recovery | Purification (X) |
| Crude Extract | 400 | 916.0 | 0.44 | --- | --- |
| Benz-Aff Chrom | 1250 | 97.3 | 12.85 | 312.5 | 29.4 |
| 1.5 M Agarose Chrom. | 855 | 29.33 | 29.15 | 68.4 | 2.3 |
| Benz-Aff Chrom | 900 | 5.85 | 153.85 | 105.3 | 5.3 |
| Phenyl-Seph. Chrom. | 208 | 0.18 | 1155.6 | 23.1 | 7.5 |
| Net Purification | | | | 52% 2644.4 | |

The enzyme obtained by this purification technique has a molecular weight of about 68,000, and is believed to be a single polypeptide chain since the electrophoretic migration was not affected by reduction with $\beta$-mercaptoethanol. When tested by analytical sodium dodecylsufate-polyacrylamide gel electrophoresis, a single protein band was observed. Furthermore, this enzyme differs from other coagulation enzymes in that it activates Factor X. By suspending the purified cancer procoagulant in Freund's adjuvant and injecting it into goats, it has been possible to obtain procoagulant specific antibody which results in a single immunoprecipitin band upon electrophoresis. This antibody can be purified by techniques such as immunoaffinity chromatography and used in the determination of cancer procoagulant in body fluids by conventional RIA or enzyme immunoassay diagnostic protocols, as will now be illustrated by Examples I-IV below.

EXAMPLE I

CANCER PROCOAGULANT ANTIGEN

Purified cancer procoagulant antigen was obtained from rabbit V2 carcinoma, human amnion-chorion tissue or other cellular sources, according to procedures described above.

Briefly, tissue, e.g., surgically removed rabbit V2 carcinoma, was extracted in 3 changes of veronal buffer, the extracts were pooled and concentrated 10-fold and used as a source of cancer procoagulant antigen. The original purification technique followed the 4 step chromatographic procedure described above. It involved benzamidine-Sepharose® affinity chromatography, 1.5 M agarose gel filtration column chromatography, a second benzamidine-Sepharose® affinity chromatography column and a phenyl-Sepharose® hydrophobic affinity chromatography column step. The protein purified by this method had all of the proper enzymatic and chemical characteristics of cancer procoagulant and was used as an antigen to immunize a goat by standard techniques described in Example II below. The partially purified goat antibody preparation was coupled to cyanogen bromide activated Sepharose® and a 1.5 x 20 cm immunoaffinity chromatography column was prepared.

In the second purification technique, the extract sample was applied to the immunoaffinity resin in 20 mM veronal buffer, the column was placed on a rotating wheel and allowed to rotate overnight so that the sample and resin were thoroughly mixed. The next morning the column was allowed to settle and the column was washed with 20 mM veronal buffer until all unbound protein was washed off the column (the absorption at 280 nm is the same as that of the buffer); this required from 250-350 ml of buffer. The column was washed with 100 ml of 5% deoxycholate dissolved in 20 mM veronal buffer [deoxycholate should be recrystallized from acetone:water (3:1)] followed by 3-4 column volumes of 20 mM veronal buffer. This removed all adsorbed proteins from the column. The column was eluted with 100 ml of 3 M NaSCN followed by 50-100 ml of veronal buffer. The eluate was dialyzed immediately against 20 mM Bis-Tris propane buffer (pH 6.5) at 5° overnight. The dialyzed eluate was concentrated on an Amicon PM10

7

ultrafiltration membrane and assayed for activity as described below. Every third or fourth run the column was cleaned with 5 M NaSCN and reequilibrated with veronal buffer. This immunoaffinity procedure removed the majority of contaminating proteins from the cancer procoagulant sample.

A p-chloromercurial benzoate (PCMB) organomercurial Agarose column (Affi-gel 501) was purchased from Bio-Rad. The column was prepared according to the Bio-Rad technical information. The column was equilibrated in 20 mM Bis-Tris propane buffer (pH 6.5). The sample was applied to the column and washed slowly onto the column with 20 mM Bis-Tris propane buffer. The column was allowed to stand for 1 hr at 4°C and washed slowly overnight with 20 mM Bis-Tris propane buffer. When the absorption at 280 nm was the same as that of the Bis-Tris propane buffer, the column was washed with about 50 ml of 1 M urea and 1% Tween in water and followed by enough 20 mM Bis-Tris propane buffer to completely remove all residual Tween-urea from the column. The column was eluted with $HgCl_2$ or glutathione, and each elution was dialyzed immediately in 20 mM Bis-Tris propane buffer at 4°C overnight with several changes of buffer. The samples were concentrated on a PM10 ultrafiltration membrane and checked for activity as described above. The purified samples from the goat immunoaffinity column and the PCMB affinity column were evaluated by SDS-polyacrylamide gel electrophoresis and the protein content of each sample was determined with the Lowry protein determination. The activity in the samples was preserved by making them 1 mM with $HgCl_2$ which will inhibit and preserve the activity for later use.

EXAMPLE II

ANTI-CANCER PROCOAGULANT GOAT IgG

One hundred micrograms of purified CP was emulsified in an equal volume of complete Freund's adjuvant and injected subcutaneously in multiple sites along the goat's spine. Booster immunizations were made at 3 week intervals by suspending 30-50 $\mu$g of purified CP in equal volume of incomplete Freund's adjuvant and injecting the goat in the same way. Blood samples were obtained by jugular vein venipuncture at monthly intervals and tested for antibody by crossed immunodiffusion. After 4 months, an antibody titer of 1:16 was reached. This level of antibody has been sustained for a minimum of 12 months. The goat antibody (a polyclonal 1gG immunoglobulin) was partially purified from goat serum by ammonium sulfate precipitation and DEAE-cellulose ion exchange chromatography by standard techniques. The partially purified antibody was found to contain antibodies to rabbit serum proteins, probably minor contaminants from the purified CP preparations of rabbit V2 carcinoma. To remove these contaminating antibodies, rabbit serum was coupled to cyanogen bromide activated Sepharose to form a normal rabbit serum protein affinity column, and the partially purified goat antibody preparation was passed over the normal rabbit serum column to remove the contaminating antibodies. The resulting goat IgG preparation was free of cross reacting antibodies with normal rabbit serum. These partially purified goat antibodies were used for immunoaffinity chromatography and in the immunoassay system.

EXAMPLE III

MONOCLONAL ANTIBODIES

Using the second purification technique described above, mice were immunized with purified CP to raise B cell antibodies as described by Yelton et al, in Monoclonal Antibodies (Kennett et al, editors) Plenum Press (New York), 1980, pgs. 3-17, although other means of raising hybridoma antibodies may also be employed. Briefly, 40 $\mu$g of purified antigen were suspended in an equal volume of complete Freund's adjuvant and injected subcutaneously into Balb/C mice. This was followed by 2 injections of 35 and 10 $\mu$g amounts of antigen suspended in incomplete Freund's adjuvant and injected subcutaneously at monthly intervals. Three weeks after the last subcutaneous immunization, 3 intraperitoneal immunizations of 10 $\mu$g, 70 $\mu$g and 70 $\mu$g of antigen in saline were administered intraperitoneally at 3 day intervals, 2 weeks later a blood sample was obtained by retroorbital bleeding and tested for serum antibody by crossed immunodiffusion; having confirmed the presence of an antibody, a last intraperitoneal immunization (40 $\mu$g) was administered, and 3 days later the animals were sacrificed. The spleen lymphocytes were removed and hybridized with P3/X 63AG8.653 variant of the mouse myeloma cell line with 50% polyethylene glycol. Hybrid cells were plated in a 96 well microtiter plate with $2 \times 10^6$ normal murine spleen cells as a feeder layer, and unhybridized myeloma cells were eliminated by growing the cultures in HAT medium for 4 weeks. An ELISA was used to screen the medium from the microtiter wells for antibody producing cells. In this assay purified antigen was adsorbed to the surface of the microtiter wells, the wells were blocked with 2% BSA, and media was incubated in the wells for 1 hr at 37°C, and an alkaline phosphatase labeled rabbit antimouse immunoglobulin preparation was added to identify the antibodies that had attached to the antigen. Positive wells were expanded in the presence of $2 \times 10^6$ normal spleen cells. Expanded wells were retested and positive wells were cloned 2 more times at low density to obtain clean and stable populations of hybrid cells for use in the experiments. Three clones were identified, each clone produced IgMK antibodies to cancer procoagulant antigen.

The IgM samples obtained from the hybrid cells (either as medium from tissue cultured cells or ascites fluid) contained procoagulant activity. In a representative experiment, Balb/C mice were injected with 0.5 ml of pristane to desensitize their immune system. Three weeks later, the mice received $2 \times 10^6$ hybridoma cells intraperitoneally, and ascites fluid was drained 3 or 4 times at 2 day intervals from the mice by intraperitoneal needle stick until the mice died. Ascites fluid was assayed for procoagulant activity, activity in factor VII-depleted plasma and inhibition by mercury and the procoagulant activity was tentatively characterized as that of cancer procoagulant. Since cancer procoagulant is believed to be an oncofetal antigen, and hybrid cells are developed from a malignant cell line (the myeloma variant), it is understandable how the antigen could be associated with the hybrid cells. Therefore, it is also probable that the IgM antibody is bound to the antigen in the ascites fluid, rendering it immunologically unreactive in the assay system. Therefore, it was necessary to separate the antigen from the antibody so that the antibody was rendered immunologically reactive to antigen in other samples. The ascites fluid was made 3 M with urea and applied to a 1 x 90 cm 1.5 M agarose gel filtration column that was preequilibrated in 3 M urea. The sample was eluted from the column in 3 M urea and the first peak (void volume) was assayed for IgM and procoagulant activity; it was free of procoagulant activity and contained all of the IgM. A second peak from the column contained procoagulant activity and no IgM . Fractions from the first peak were pooled, dialyzed against at least 3 changes of 5 mM Tris-HCl buffer (pH 7.5), the sample was concentrated over an Amicon XM50 ulrafiltration membrane, and refrigerated overnight in a centrifuge tube. The next morning, a precipitate had formed in the test tube, it was removed by centrifugation and resuspended in PBS. The resuspended precipitate sample was found to contain the immunoreactive IgM fraction, and a small amount had remained behind in the supernatant. This purified IgM was assayed against purified antigen, using 2% normal human serum as a control blank and gave a sample to blank ratio of from 10 to 20. The unpurified ascites gave a sample to background ratio of from 2 to 4, the supernatant gave a sample to background ratio of 6 to 10. This purified IgM was then used in the immunoassay. There are other methods for dissociating antigen-antibody complexes so they can be separated. Such methods may include higher concentrations of urea, low pH (pH 2-3.5), 5M guanidine-HCl, high pH (pH 10.5-12) and combinations of dissociating agents and pH adjustment. All such methods for separating antibody-cancer procoagulant antigen complexes are included within the perview of this application.

## EXAMPLE IV

## IMMUNOASSAY

Two separate immunoassays for the quantification of cancer procoagulant were developed.

The first immunoassay system was a direct ELISA in which antigen was adsorbed to the surface of the wells in a 96 well Immulon I microtiter plate at room temperature for 2 hrs, the well was rinsed with phosphate buffered Tween-20, the open sites on the wells were blocked with 2% normal human serum in phosphate buffer at 37°C for 1 hr, and the wells were washed 3 times with 20 mM phosphate buffer (pH 7.5) containing 0.05% Tween®-20. Purified IgM antibody was diluted 1:200 in phosphate buffer and 50 $\mu$l was added to each well and incubated at 37°C for 1 hr. The wells were washed 3 times with phosphate buffer containing .05% Tween® (PTB). One to 1000 dilution of alkaline phosphatase labeled rabbit antimouse IgM antibody was added to each well, incubated for 1 hr at 37°C, the wells were washed with PTB and 100 $\mu$l of p-nitrophenyl phosphate (5 mg/ml) in 10% diethanolamine buffer (pH 9.8) containing 0.1 mg $MgCl_2.6H_2O$/ml and 0.2% $NaN_3$ and incubated at 37°C for from 45 to 90 min (until color intensity is adequate to read), and then the plate was read on a Dynatech microtiter plate reader which measured absorbance at 405 nm.

In a second ELISA procedure (a sandwich or double antibody ELISA), the Immunolon I microtiter plate was coated with 1 to 40,000 dilution of partially purified goat IgG and incubated for 2 hrs at 25°C, the wells were washed once with phosphate buffered saline (PBS) and open sites in the wells were blocked with 2% human serum in phosphate buffer. The wells were washed 3 times with PTB, 50 $\mu$l of the antigen sample (usually diluted 1:2 with PTB + 0.15 M NaCl) was added to each well and incubated at 25°C for 2 h, the wells were washed again with PTB, 50 μl of 1 to 200 dilution of IgM in PBT was added and incubated at 25°C for 2 hrs, and the amount of IgM was measured as described above.

Both of these assays were used to measure purified antigen, purified antigen added to normal human serum, serum from cancer patients, extracts of tumors and other biological samples. The first assay worked better for more purified samples, the second assay worked better for samples like serum and other samples that contain a large number of other proteins that competed with the antigen for binding to the surface of the well because the antigen was absorbed out of the biological sample onto the goat antibody, and the monoclonal antibody was used to quantitate the amount of antigen. Both ELISA procedures were able to detect 10 ng of purified antigen.

The ELISA method is one of a variety of immunoassay techniques that could be employed to assay for cancer procoagulant antigen in biological samples. Other methods include radioimmunoassay, immunoinhibition assay, immunofluorescent assay and immunoprecipitation assay; all such assays that include the use of an antibody to quantitate the cancer procoagulant antigen should be construed to be included under the description of the assay.

To prove the effectiveness of the ELISA according to the present invention to diagnose cancer, a large number of serum samples from documented cancer patients and non-cancer controls were tested in blind runs. Table 1 contains the data obtained from the immunoassays conducted and includes the categories of cancers evaluated, the number of individuals studied, the number of samples correctly identified, and the percentage of correctly identified samples.

## TABLE I

| Tumor Type or Site | Total No. | Correct No. | % Correct |
|---|---|---|---|
| Gastrointestinal | 33 | 31 | 94 |
| Respiratory | 29 | 24 | 83 |
| Breast | 35 | 26 | 74 |
| Prostate | 6 | 6 | 100 |
| Bone | 4 | 4 | 100 |
| Lymphoma | 6 | 5 | 83 |
| Pancreatic | 8 | 7 | 88 |
| Other | 12 | 9 | 75 |

Control Samples:

| | Total No. | Correct No. | % Correct |
|---|---|---|---|
| Normal Controls | 107 | 98 | 92 |
| Benign Disease Controls | 10 | 10 | 100 |

**Claims**

**Claims for the following Contracting States : BE, CH, DE, FR, GB, IT, LI, LU, NL, SE**

1. Dissociated and purified monoclonal antibody to cancer procoagulant, a cysteine protease associated with malignant cells and having procoagulant activity, said monoclonal antibody having immunological reactivity to cancer procoagulant and being substantially free of procoagulant activity.

2. A method for producing immunologically reactive monoclonal antibody to cancer procoagulant, a cysteine protease associated with malignant cells and having procoagulant activity, said method comprising the steps of:
   (a) immunizing an animal with cancer procoagulant antigen;
   (b) fusing spleen cells from said immunized animal with myeloma cells to form hybrid cells;
   (c) culturing said hybrid cells in a selective medium;
   (d) testing for the presence of the desired antibody;
   (e) cloning cells producing the desired antibody; and
   (f) isolating the desired antibody, the method being characterized by subjecting medium from said cells producing the desired antibody obtained in step (e) to the step of dissociating said antibody from cancer procoagulant antigen produced by the hybrid cells used for the production of said antibody, prior to isolating the desired antibody in step (f), whereby the antibody obtained in step (f) is immunologically reactive to, and substantially free of, cancer procoagulant.

3. The method of Claim 2, wherein the step of dissociating comprises exposure to a dissociating condition selected from the group consisting of a dissociating agent, a pH adjustment and combinations of dissociating agents and pH adjustments.

4. The method of Claim 3, wherein said dissociating agent is selected from the group consisting of at least about 3M urea and about 5M guanidine.

5. The method of Claim 3, wherein said pH adjustment is selected from the group consisting of pH between about 2.0 and about 3.5 and pH between about 10.5 and about 12.0.

6. The method of Claim 2, wherein the step of isolating the desired antibody comprises chromatographic separation.

7. A reagent for detection of cancer procoagulant comprising immunologically reactive monoclonal antibody according to Claim 1 or produced by the method of any one of Claims 2-6.

8. A reagent according to Claim 7, comprising also polyclonal antibodies to cancer procoagulant.

9. A method for detecting for the presence of cancer procoagulant in a biological sample of a patient, comprising contacting said biological sample with a reagent according to Claim 7 or Claim 8, and detecting the presence or absence of an immunological reaction.

10. A method according to Claim 9, wherein said biological sample consists of a biological fluid removed from said patient.

11. A method according to Claim 10, wherein said biological fluid is serum, plasma or urine.

**Claims for the following Contracting State : AT**

1. A method for producing monoclonal antibody to cancer procoagulant, a cysteine protease associated with malignant cells and having procoagulant activity, comprising the steps of:
   (a) immunizing an animal with cancer procoagulant antigen;
   (b) fusing spleen cells from said immunized animal with myeloma cells to form hybrid cells;
   (c) culturing said hybrid cells in a selective medium;
   (d) testing for the presence of the desired antibody;
   (e) cloning cells producing the desired antibody;

11

(f) isolating the desired antibody;
    the method being characterized by
    subjecting medium from said cells producing the desired antibody obtained in step (e) to the step of dissociating said antibody from cancer procoagulant antigen produced by the hybrid cells used for the production of said antibody, prior to isolating the desired antibody in step (f), whereby the antibody obtained in step (f) is immunologically active.

2. The method of Claim 1, wherein the step of dissociating comprises exposure to a dissociating condition selected from the group consisting of a dissociating agent, a pH adjustment and combinations of dissociating agents and pH adjustments.

3. The method of Claim 2, wherein said dissociating agent is selected from the group consisting of at least about 3M urea and at about 5M guanidine.

4. The method of Claim 2, wherein said pH adjustment is selected from the group consisting of pH between about 2.0 and about 3.5 and pH between about 10.5 and about 12.0.

5. The method of Claim 1, wherein the step of isolating the desired antibody comprises chromatographic separation.

6. A reagent for detection of cancer procoagulant comprising immunologically reactive monoclonal antibody produced by the method of any one of Claims 1-5.

7. A reagent according to Claim 6, comprising also polyclonal antibodies to cancer procoagulant.

8. A method for detecting for the presence of cancer procoagulant in a biological fluid removed from a patient, comprising contacting said biological fluid with a reagent according to claims 6 or 7 and detecting the presence or absence of an immunological reaction.

9. A method according to Claim 8, wherein said biological fluid is serum, plasma or urine.

**Revendications**

**Revendications pour les Etats contractants suivants : BE, CH, DE, FR, GB, IT, LI, LU, NL, SE**

1. Anticorps monoclonal, dissocié et purifié, à l'encontre de procoagulant du cancer, une protéase de la cystéine associée à des cellules malignes et ayant une activité procoagulante, ledit anticorps monoclonal ayant une réactivité immunologique à l'encontre du procoagulant du cancer et étant notablement exempt d'activité procoagulante.

2. Procédé de préparation d'un anticorps monoclonal réactif dans le domaine immunologique à l'encontre de procoagulant du cancer, une protéase de la cystéine associée à des cellules malignes et ayant une activité procoagulante, ledit procédé comprenant les étapes suivantes :
    a) immunisation d'un animal à l'aide d'un antigène de procoagulant du cancer;
    b) fusion de cellules de la rate provenant dudit animal immunisé avec des cellules de myélome, pour obtenir des cellules hybrides;
    c) culture de ces cellules hybrides dans un milieu sélectif;
    d) contrôle de la présence de l'anticorps désiré;
    e) clonage des cellules produisant l'anticorps désiré; et
    f) isolement de l'anticorps désiré, ce procédé étant caractérisé par le fait que l'on soumet le milieu provenant desdites cellules produisant l'anticorps désiré, obtenu dans l'étape e) à l'étape de dissociation dudit anticorps de l'antigène de procoagulant du cancer produit par les cellules hybrides utilisées à la production dudit anticorps, avant l'isolement de l'anticorps désiré dans l'étape f), grâce à quoi l'anticorps obtenu à l'étape f) est capable de réactivité immunologique à l'encontre de procoagulant du cancer, dont il est notablement exempt.

3. Procédé selon la revendication 2, dans lequel l'étape de dissociation consiste en une exposition à des conditions de dissociation choisies parmi le groupe constitué par un agent de dissociation, un réglage du $p_H$ et des combinaisons d'agents de dissociation et de réglages du $p_H$.

4. Procédé selon la revendication 3, dans lequel l'agent de dissociation est choisi dans le groupe constitué par au moins de l'urée 3M environ et de la guanidine 5M environ.

5. Procédé selon la revendication 3, dans lequel le réglage du pH est choisi dans la plage constituée par les $p_H$ compris entre 2,0 et 3,5 environ et dans la plage constituée par les $p_H$ compris entre 10,5 et 12,0 environ.

6. procédé selon la revendication 2, dans lequel l'étape d'isolement de l'anticorps désiré comporte une séparation chromatographique.

7. Réactif en vue de la détection de procoagulant du cancer comprenant un anticorps monoclonal réactif dans le domaine immunologique, conformément à la revendication 1 ou préparé par le procédé selon l'une quelconque des revendications 2 à 6.

8. Réactif selon la revendication 7, comprenant aussi des anticorps polyclonaux à l'encontre de procoagulant du cancer.

9. Procédé de détection de la présence de procoagulant du cancer dans un échantillon biologique d'un patient, comprenant les étapes de mise en contact dudit échantillon biologique avec un réactif selon la revendication 7 ou la revendication 8, et de détection de la présence ou de l'absence de réaction immunologique.

10. Procédé selon la revendication 9, dans lequel ledit échantillon biologique consiste en un fluide biologique extrait dudit patient.

11. Procédé selon la revendication 10, dans lequel ledit fluide biologique est du sérum, du plasma ou de l'urine.

**Revendications pour l'Etat contractant suivant : AT**

1. Procédé de préparation d'un anticorps monoclonal réactif dans le domaine immunologique à l'encontre de procoagulant du cancer, une protéase de la cystéine associée à des cellules malignes et ayant une activité procoagulante, ledit procédé comprenant les étapes suivantes :
   a) immunisation d'un animal à l'aide d'un antigène de procoagulant du cancer;
   b) fusion de cellules de la rate provenant dudit animal immunisé avec des cellules de myélome, pour obtenir des cellules hybrides;
   c) culture de ces cellules hybrides dans un milieu sélectif;
   d) contrôle de la présence de l'anticorps désiré;
   e) clonage des cellules produisant l'anticorps désiré; et
   f) isolement de l'anticorps désiré, ce procédé étant caractérisé par le fait que l'on soumet le milieu provenant desdites cellules produisant l'anticorps désiré, obtenu dans l'étape e) à l'étape de dissociation dudit anticorps de l'antigène de procoagulant du cancer produit par les cellules hybrides utilisées à la production dudit anticorps, avant l'isolement de l'anticorps désiré dans l'étape f), grâce à quoi l'anticorps obtenu à l'étape f) est capable de réactivité immunologique.

2. Procédé selon la revendication 1, dans lequel l'étape de dissociation consiste en une exposition à des conditions de dissociation choisies parmi le groupe constitué par un agent de dissociation, un réglage du $p_H$ et des combinaisons d'agents de dissociation et de réglages du $p_H$.

3. Procédé selon la revendication 2, dans lequel l'agent de dissociation est choisi dans le groupe constitué par au moins de l'urée 3M environ et de la guanidine 5M environ.

**4.** Procédé selon la revendication 2, dans lequel le réglage du $p_H$ est choisi dans la plage constituée par les $p_H$ compris entre 2,0 et 3,5 environ et dans la plage constituée par les $p_H$ compris entre 10,5 et 12,0 environ.

**5.** procédé selon la revendication 1, dans lequel l'étape d'isolement de l'anticorps désiré comporte une séparation chromatographique.

**6.** Réactif en vue de la détection de procoagulant du cancer comprenant un anticorps monoclonal réactif dans le domaine immunologique, préparé par le procédé selon l'une quelconque des revendications 1 à 5.

**7.** Réactif selon la revendication 6, comprenant aussi des anticorps polyclonaux à l'encontre de procoagulant du cancer.

**8.** Procédé de détection de la présence de procoagulant du cancer dans un fluide biologique extrait d'un patient, comprenant les étapes de mise en contact dudit fluide biologique avec un réactif selon l'une des revendications 6 ou 7, et de détection de la présence ou de l'absence de réaction immunologique.

**9.** Procédé selon la revendication 8, dans lequel ledit fluide biologique est du sérum, du plasma ou de l'urine.

**Patentansprüche**

**Patentansprüche für folgende Vertragsstaaten : BE, CH, DE, FR, GB, IT, LI, LU, NL, SE**

**1.** Dissoziierter und gereinigter monoklonaler Antikörper gegen Krebsprokoagulans, das heißt gegen eine Zysteinprotease, die mit malignen Zellen assoziiert ist und Prokoagulanswirkung aufweist, wobei der genannte monoklonale Antikörper immunologische Reaktivität auf Krebsprokoagulans aufweist und im wesentlichen frei von Prokoagulanswirkung ist.

**2.** Verfahren zur Herstellung von immunologisch reaktivem monoklonalem Antikörper gegen Krebsprokoagulans, das heißt gegen eine Zysteinprotease, die mit malignen Zellen assoziiert ist und Prokoagulanswirkung aufweist, wobei das genannte Verfahren folgende Schritte umfaßt:
(a) Immunisieren eines Tiers mit Krebsprokoagulansantigen;
(b) Verschmelzen von Milzzellen aus genanntem immunisiertem Tier mit Myelomazellen, um Hybridzellen zu bilden;
(c) Kultivieren der genannten Hybridzellen in einem selektiven Medium;
(d) Testen in bezug auf Gegenwart des gewünschten Antikörpers;
(e) Klonen von Zellen, welche den gewünschten Antikörper erzeugen; und
(f) Isolieren des gewünschten Antikörpers, wobei das Verfahren dadurch gekennzeichnet ist, daß Medium aus den genannten Zellen, welche den gewünschten, in Schritt (e) erhaltenen Antikörper erzeugen, dem Schritt des Dissoziierens des genannten Antikörpers vom Krebsprokoagulansantigen unterworfen wird, das von den zur Herstellung des genannten Antikörpers eingesetzten Hybridzellen hergestellt wird, vor dem Isolieren des gewünschten Antikörpers in Schritt (f), wodurch der in Schritt (f) erhaltene Antikörper immunologisch reaktiv auf und im wesentlichen frei von Krebsprokoagulans ist.

**3.** Verfahren nach Anspruch 2, worin der Schritt des Dissoziierens das Aussetzen an eine Dissoziierungsbedingung umfaßt, die aus der Gruppe ausgewählt ist, die aus einem Dissoziierungsagens, einer pH-Einstellung und Kombinationen aus Dissoziierungsagenzien und pH-Einstellungen besteht.

**4.** Verfahren nach Anspruch 3, worin das genannte Dissoziierungsagens aus der Gruppe ausgewählt ist, die aus zumindest etwa 3M Harnstoff und etwa 5M Guanidin besteht.

**5.** Verfahren nach Anspruch 3, worin die genannte pH-Einstellung aus der Gruppe ausgewählt ist, die aus einem pH-Wert zwischen etwa 2,0 und etwa 3,5 und einem pH-Wert zwischen etwa 10,5 und etwa 12,0 besteht.

14

**6.** Verfahren nach Anspruch 2, worin der Schritt des Isolierens des gewünschten Antikörpers chromatographische Trennung umfaßt.

**7.** Reagens zum Nachweis von Krebsprokoagulans, das immunologisch reaktiven monoklonalen Antikörper nach Anspruch 1 oder Antikörper, der durch das Verfahren nach einem der Ansprüche 2 bis 6 hergestellt ist, umfaßt.

**8.** Reagens nach Anspruch 7, das auch polyklonale Antikörper gegen Krebsprokoagulans umfaßt.

**9.** Verfahren zum Nachweis der Gegenwart von Krebsprokoagulans in einer biologischen Probe eines Patienten, welches das In-Kontakt-bringen der genannten biologischen Probe mit einem Reagens nach Anspruch 7 oder 8 und das Nachweisen der Gegenwart oder Abwesenheit einer immunologischen Reaktion umfaßt.

**10.** Verfahren nach Anspruch 9, worin die genannte biologische Probe aus einem vom genannten Patienten entnommenen biologischen Fluid besteht.

**11.** Verfahren nach Anspruch 10, worin das genannte biologische Fluid Serum, Plasma oder Harn ist.

**Patentansprüche für folgenden Vertragsstaat: AT**

**1.** Verfahren zur Herstellung von monoklonalem Antikörper gegen Krebsprokoagulans, das heißt gegen eine Zysteinprotease, die mit malignen Zellen assoziiert ist und Prokoagulanswirkung aufweist, welches folgende Schritte umfaßt:
(a) Immunisieren eines Tiers mit Krebsprokoagulansantigen;
(b) Verschmelzen von Milzzellen aus genanntem immunisiertem Tier mit Myelomazellen, um Hybridzellen zu bilden;
(c) Kultivieren der genannten Hybridzellen in einem selektiven Medium;
(d) Testen in bezug auf Gegenwart des gewünschten Antikörpers;
(e) Klonen von Zellen, welche den gewünschten Antikörper erzeugen;
(f) Isolieren des gewünschten Antikörpers, wobei das Verfahren dadurch gekennzeichnet ist, daß Medium aus den genannten Zellen, welche den gewünschten, in Schritt (e) erhaltenen Antikörper erzeugen, dem Schritt des Dissoziierens des genannten Antikörpers vom Krebsprokoagulansantigen unterworfen wird, das von den zur Herstellung des genannten Antikörpers eingesetzten Hybridzellen hergestellt wird, vor dem Isolieren des gewünschten Antikörpers in Schritt (f), wodurch der in Schritt (f) erhaltene Antikörper immunologisch wirksam ist.

**2.** Verfahren nach Anspruch 1, worin der Schritt des Dissoziierens das Aussetzen an eine Dissoziierungsbedingung umfaßt, die aus der Gruppe ausgewählt ist, die aus einem Dissoziierungsagens, einer pH-Einstellung und Kombinationen aus Dissoziierungsagenzien und pH-Einstellungen besteht.

**3.** Verfahren nach Anspruch 2, worin das genannte Dissoziierungsagens aus der Gruppe ausgewählt ist, die aus zumindest etwa 3M Harnstoff und etwa 5M Guanidin besteht.

**4.** Verfahren nach Anspruch 2, worin die genannte pH-Einstellung aus der Gruppe ausgewählt ist, die aus einem pH-Wert zwischen etwa 2,0 und etwa 3,5 und einem pH-Wert zwischen etwa 10,5 und etwa 12,0 besteht.

**5.** Verfahren nach Anspruch 1, worin der Schritt des Isolierens des gewünschten Antikörpers chromatographische Trennung umfaßt.

**6.** Reagens zum Nachweis von Krebsprokoagulans, das immunologisch reaktiven monoklonalen Antikörper umfaßt, welcher durch das Verfahren nach einem der Ansprüche 1 bis 5 hergestellt ist.

**7.** Reagens nach Anspruch 6, das auch polyklonale Antikörper gegen Krebsprokoagulans umfaßt.

EP 0 162 977 B1

8. Verfahren zum Nachweis der Gegenwart von Krebsprokoagulans in einem von einem Patienten entnommenen biologischen Fluid, welches Verfahren das In-Kontakt-bringen des genannten biologischen Fluids mit einem Reagens nach Anspruch 6 oder 7 und das Nachweisen der Gegenwart oder Abwesenheit einer immunologischen Reaktion umfaßt.

9. Verfahren nach Anspruch 8, worin das genannte biologische Fluid Serum, Plasma oder Harn ist.

16